**Europäisches Patentamt**

**European Patent Office**

**Office européen des brevets**

(19)

(11) Veröffentlichungsnummer : **0 024 504**
**B1**

(12) # EUROPÄISCHE PATENTSCHRIFT

(45) Veröffentlichungstag der Patentschrift :
30.03.83

(21) Anmeldenummer : **80103665.8**

(22) Anmeldetag : **27.06.80**

(51) Int. Cl.³ : **A 61 N   5/10, A 61 B   6/08**

(54) **Strahlentherapiegerät mit zwei Lichtvisieren.**

(30) Priorität : **03.07.79 DE 2926873**

(43) Veröffentlichungstag der Anmeldung :
**11.03.81 Patentblatt 81/10**

(45) Bekanntmachung des Hinweises auf die Patenterteilung : **30.03.83 Patentblatt 83/13**

(84) Benannte Vertragsstaaten :
**DE FR GB SE**

(56) Entgegenhaltungen :
**DD A 23 132**
**DE B 2 200 816**
**DE C 954 449**
**FR A 1 021 814**
**FR A 2 137 159**
**US A 2 204 465**
**US A 2 474 422**
**US A 2 887 586**
**US A 3 803 418**

(73) Patentinhaber : **SIEMENS AKTIENGESELLSCHAFT**
**Berlin und München Wittelsbacherplatz 2**
**D-8000 München 2 (DE)**

(72) Erfinder : **Heinz, Lothar**
**Effeltricher Strasse 5**
**D-8524 Neunkirchen a. Brand (DE)**

EP 0 024 504 B1

### Strahlentherapiegerät mit zwei Lichtvisieren

Die Erfindung bezieht sich auf ein Strahlentherapiegerät zur Bestrahlung von Patienten, mit einer Strahlenblende, mit Lichtvisieren für die Projektion von die Geräteeinstellung markierenden Koordinaten auf die Oberfläche des zu bestrahlenden Objektes und mit zwei je einen zweidimensionalen Lichtfächer projizierenden Lichtvisieren, die in der Weise am Strahlentherapiegerät befestigt sind, daß sich die Ebenen der beiden Lichtfächer längs der Symmetrieachse der Strahlenblende schneiden und die Projektionsrichtungen der Lichtfächer über den gesamten Öffnungswinkel eines jeden der beiden Lichtfächer hinweg einen Winkel sowohl mit der Symmetrieachse als auch mit senkrecht zur Symmetrieachse ausgerichteten Ebenen bilden.

Bei Röntgenuntersuchungsgeräten ist es durch die US-Patentschriften 22 04 465, 24 74 422 und 28 87 586 bekannt, im Röntgenstrahlenkegel zwischen der Strahlenquelle und der Strahlenblende einen um ca. 45° gegenüber der Symmetrieachse der Strahlenblende geneigten Spiegel anzuordnen. Durch diesen Spiegel wird das Licht einer seitlich des Spiegels angeordneten Lichtquelle durch die Ausblendöffnung der Primärstrahlenblende hindurch auf die Oberfläche des Untersuchungsobjektes geworfen. Der Lichtkegel wird dabei in der gleichen Weise von den Blendenplatten der Primärstrahlenblende begrenzt wie die Röntgenstrahlung. Bei diesen sog. Vollfeldlichtvisieren leuchtet die Lichtquelle genau das gleiche Feld aus, das auch von der Röntgenstrahlung bei eingeschalteter Röntgenstrahlenquelle getroffen würde. Solche Lichtvisiere erleichtern wegen des auf der Hautoberfläche sichtbaren beleuchteten Feldes die Einstellung des Strahlenfeldes auf den Untersuchungsbereich des Patienten beträchtlich. Sie werden häufig auch mit einem Fadenkreuz ausgerüstet, um die Symmetrieachse der Primärstrahlenblende auf der Hautoberfläche zu markieren.

Die USA-Patentschrift 28 87 586 offenbart zusätzlich einen seitlich der Strahlenblende angeordneten Projektor, der eine Skala auf den Untersuchungstisch projiziert. Der Kreuzungspunkt dieser Skala mit einem auf dem Untersuchungstisch angebrachten Streifen ist ein Maß für den Abstand des Fokus von der Tischoberfläche. Der Abstand der Hautoberfläche des Patienten vom Fokus ist mit dieser Einrichtung jedoch nicht feststellbar. Sie muß separat mit dem Bandmaß ermittelt werden.

Durch die US-PS 38 03 418 ist es auch bekannt, einen Projektor in die Rotationsachse, um die das Röntgenuntersuchungsgerät gedreht werden kann, einzubauen und mit ihm einen fadenkreuzförmigen Lichtstrahl längs der Rotationsachse des Gerätes auszustrahlen. Aus dem zu bestrahlenden Objekt zeigt das Fadenkreuz den Punkt an, der auf der Rotationsachse liegt. Bei der Bestimmung des strahlentherapeutisch vor allem interessierenden Isozentrums wird es jedoch als nachteilig empfunden, daß das beispielsweise auf den Schädel eines Patienten projizierte Fadenkreuz für die Rotationsachse manchmal weit entfernt von dem beispielsweise im Körperstamm befindlichen Krankheitsherd ist.

Bei einem Strahlentherapiegerät ist es auch bekannt, eine das Fadenkreuz des Lichtvisiers der Strahlenblende schneidende Skala mittels eines zusätzlichen, seitlich der Primärstrahlenblende angeordneten Projektors auf die Hautoberfläche des Patienten zu projizieren. Dieser Skala sind Zahlenwerte zugeordnet, die im Schnittpunkt des auf die Hautoberfläche projizierten Fadenkreuzes des Lichtvisiers den Abstand der Hautoberfläche vom Fokus angeben. Bei dieser sehr praktischen Angabe des Fokus-Haut-Abstandes wird es jedoch als nachteilig empfunden, daß sie nur für Röntgenuntersuchungsgeräte und für Strahlentherapiegeräte, welche mit Röntgenstrahlen oder Gammastrahlen arbeiten, benutzbar ist. Bei Strahlentherapiegeräten, die mit einer Strahlenart geringerer Durchdringungsfähigkeit arbeiten, wie beispielsweise mit beschleunigten Elektronen, ist diese Einrichtung wegen der Eigenabsorption des im Strahlengang vorzusehenden Ablenkspiegels des Lichtvisiers nicht brauchbar.

Durch die französische Patentschrift 10 21 814 ist eine Einrichtung mit zwei einen fächerförmigen Lichtkegel aussendenden Lichtvisieren bekannt, die so neben einer Primärstrahlenblende einer Röntgenröhre montiert werden können, daß sich die Lichtfächer in einer Linie durchsetzen, die mit dem Zentrahstrahl der Primärstrahlenblende übereinstimmt. Wird ein Gegenstand in nahezu beliebigem Abstand unter der Primärstrahlenblende angeordnet, so sind die beiden Lichtfächer auf seiner Oberfläche als Fadenkreuz zu erkennen. Sie markieren den Auftreffpunkt des Zentralstrahls auf diesem Gegenstand. Dieser Druckschrift ist jedoch kein Hinweis zu entnehmen, wie mit solch einer Einrichtung der Fokus-Objekt-Abstand anzuzeigen ist.

Der Erfindung liegt die Aufgabe zugrunde, einen Weg zu weisen, wie man bei Strahlentherapiegeräten, insbesondere solchen, die mit wenig durchdringender Strahlung, wie z. B. mit beschleunigten Elektronen, arbeiten, die Lage des Zentralstrahls sowie die Tiefe des Isozentrums im Körper des Patienten in einfacher Weise anzeigen kann.

Bei einem Strahlentherapiegerät der eingangs genannten Art wird daher erfindungsgemäß eine Skale mit dem einen der beiden Lichtfächer mitprojiziert, die im jeweiligen Schnittpunkt der beiden Lichtfächer mit einer senkrecht zur Symmetrieachse ausgerichteten Ebene die Entfernung zum Fokus der Strahlenquelle des Strahlentherapiegerätes bezeichnet. Bei dieser Konstruktion, die es vermeidet, daß sich irgendein Teil eines Lichtvisiers im Strahlengang des Strahlentherapiegerätes befindet und dort Strahlung absorbieren kann, kann durch die Mitprojektion einer Skala mit dem einen Lichtfächer und durch die Lage der Skala zum Schnittpunkt der Lichtfächer, der Abstand des Fokus von der Strahlenquelle unmittelbar im Schnittpunkt der Lichtfächer abgelesen werden. Da aber auch der Abstand des Fokus von der Rotationsachse des Gerätes bekannt ist, braucht nur der Zahlenwert, der abgelesen worden ist, von dem bekannten Wert des Abstandes des Fokus von der Rotationsachse abgezogen zu werden, um die Tiefe der Lage des Isozentrums unter der Hautoberfläche zu erhalten.

Relativ gute Ableseeigenschaften der Skala werden erreicht, wenn die Ebenen der beiden Lichtfächer in Ausgestaltung der Erfindung in einer senkrecht zur Symmetrieachse der Strahlenblende des Strahlentherapiegerätes ausgerichteten Ebene miteinander einen Winkel von mindestens 10°, jedoch von höchstens 170° bilden. In diesem Bereich schneiden sich die beiden Lichtfächer in einem genügend großen Winkel, so daß ihr Schnittpunkt hinreichend genau abzulesen ist.

Eine besonders brauchbare Konstruktion erhält man, wenn die Projektionsrichtung der Lichtvisiere in Ausgestaltung der Erfindung über den gesamten Öffnungswinkel des jeweiligen Lichtfächers hinweg mit einer senkrecht zur Symmetrieachse der Strahlenblende ausgerichteten Ebene einen Winkel von mindestens 20° bis höchstens 80° bilden. Wird der Winkel kleiner als 20° gewählt, so kann es vorkommen, daß die projizierten Lichtmarken durch Wölbungen der Körperoberfläche verdeckt werden. Wird der Windel größer als 80° gewählt, so verringert sich die Ablesegenauigkeit der Skala zu sehr.

In vorteilhafter Weiterbildung der Erfindung können die Ziffern der Skala im Lichtvisier in der Weise verzerrt sein, daß sie erst durch die Projektion auf eine senkrecht zur Symmetrieachse ausgerichtete Ebene entzerrt werden. Diese Maßnahme hat den Vorteil, daß auch mit einem flachen Winkel auf der meist senkrecht zur Symmetrieachse ausgerichteten Hautoberfläche des Patienten projizierte Skalenwerte unschwer abgelesen werden können.

Weitere Einzelheiten der Erfindung werden anhand eines in den Figuren dargestellten Ausführungsbeispiels näher erläutert. Es zeigen :

Figur 1 eine schaubildliche Ansicht eines Strahlentherapiegerätes,

Figur 2 eine schematische Darstellung der auf einer Oberfläche projizierten Koordinaten,

Figur 3 einen Schnitt durch eines der Lichtvisiere, und

Figur 4 einen Ausschnitt einer im Lichtvisier eingebauten Skalenschablone.

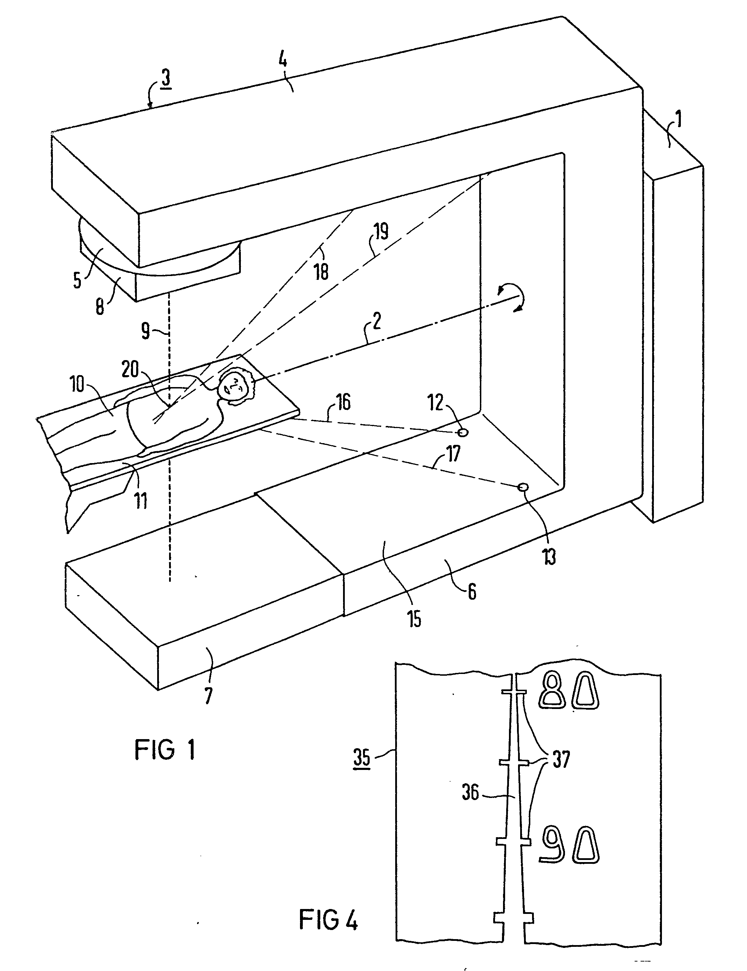

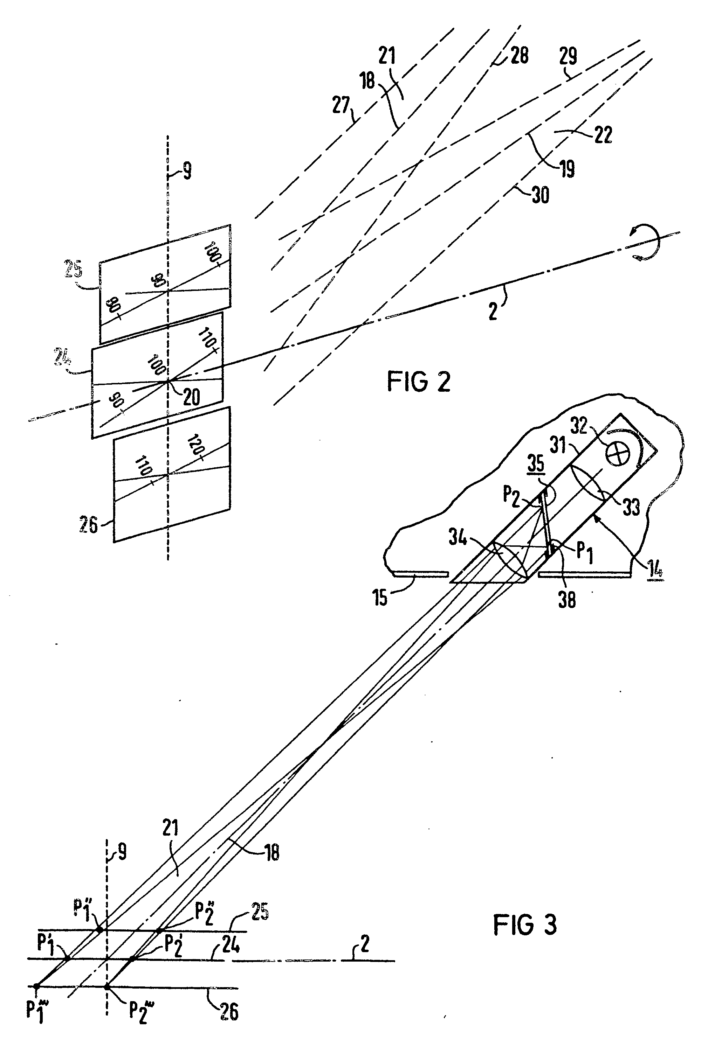

Die Figur 1 zeigt ein an einer im Fußboden verankerten Stativsäule 1 um eine horizontale Rotationsachse 2 drehbares Strahlentherapiegerät 3. Das Strahlentherapiegerät, ein Elektronenbeschleuniger, ist im wesentlichen U-förmig aufgebaut. Es trägt an seinem einen Schenkel 4 einen Strahlerkopf 5. Der andere Schenkel 6 trägt einen im Bedarfsfall ausfahrbaren Strahlenfänger 7. Im Strahlerkopf 5 befindet sich unter anderem eine Strahlenquelle (nicht dargestellt), im vorliegenden Fall ein Elektronenbeschleuniger mit einem Elektronenaustrittsfenster, und eine Strahlenblende 8. Die Symmetrieachse 9 der Strahlenblende 8, die bei symmetrischer Aufblendung mit dem Zentralstrahl des austretenden Strahlenfeldes zusammenfällt, ist in der Figur punktiert angedeutet und auf den in der Figur 1 ausgefahrenen Strahlenempfänger 7 am Ende des anderen Schenkels 6 zentriert. Das zu bestrahlende Objekt bzw. der Patient 10 befindet sich auf einer Lagerungsplatte 11 im Bereich des Strahlenfeldes. Auf der Innenseite eines jeden der Schenkel 4, 6 sind im Randbereich je zwei Lichtvisiere 12, 13, 14 (nur drei dargestellt) im Gehäuse 15 des Strahlentherapiegerätes eingebaut. Die optischen Achsen 16, 17, 18 19 dieser Lichtvisiere sind in der Figur 1 durch gestrichelte Linien angedeutet. Sie sind zum Isozentrum 20, d. h. dem Schnittpunkt der strichpunktierten Rotationsachse 2 des Strahlentherapiegerätes 3, mit der punktierten Symmetrieachse 9 der Strahlenblende 8 ausgerichtet. Die Lichtvisiere projizieren je einen ebenen Lichtfächer 21, 22 (Fig. 2, 3), der die Symmetrieachse 9 in seiner gesamten Breite schneidet.

In der Figur 2 sind als Bezugskoordinaten die strichpunktierte horizontale Rotationsachse 2 und die punktierte Symmetrieachse 9 der Strahlenblende 8 des Strahlentherapiegerätes 8, die sich beide im Isozentrum 20 schneiden, eingezeichnet. Durch dieses Isozentrum ist zur Verdeutlichung der Funktion eine senkrecht zur Symmetrieachse der Strahlenblende ausgerichtete Ebene 24 eingezeichnet. Parallel zu dieser Ebene 24 sind zu beiden Seiten zwei weitere Ebenen 25, 26 eingezeichnet. Ferner sind in der Figur 2 die beiden sich schneidenden ebenen Lichtfächer 21, 22 der beiden Lichtvisiere eines der beiden Schenkel des Strahlentherapiegerätes in gestrichelten Linien eingezeichnet. Die Lichtvisiere senden je einen durch zwei gestrichelte Linien 27, 28, 29, 30 angedeuteten zweidimensionalen Lichtfächer 21, 22 aus. Diese Lichtfächer durchsetzen einander längs der Symmetrieachse 9 der Strahlenblende 8. Die beiden Lichtfächer projizieren sich daher auf den senkrecht zur Symmetrieachse der Strahlenblende ausgerichteten Ebenen 24, 25, 26 als sich kreuzende Linien. Sie sind in den drei in der Figur 2 eingezeichneten Ebenen ausgezogen. Der Schnittpunkt der sich kreuzenden Linien kennzeichnet in jeder Ebene den Auftreffpunkt der Symmetrieachse 9 der Strahlenblende 8:

Die Figur 3 zeigt einen Schnitt durch eines der Lichtvisiere 14. Es besteht aus einem zylindrischen Gehäuse 31 mit einer eingebauten Lichtquelle 32, einer vor der Lichtquelle angeordneten Kondensorlinse 33 und einer am offenen Ende des Gehäuses 31 parallel zur Kondensorlinse 33 angeordneten Objektivlinse 34. Zwischen der Kondensorlinse und der Objektivlinse ist eine Schablone 35 mit dem den Lichtfächer 21 erzeugenden Spalt 36 und einer Skala 37 (Fig. 4) in einem Einschubrahmen 38 eingesetzt. Die Ebene der Schablone 35 ist dem Betrage nach um den gleichen Winkel, jedoch in entgegengesetzter Richtung zur optischen Achse 18 des Lichtvisiers 14 verschwenkt wie die Bildebene 24, auf der die Skala 37 abgebildet werden soll, d. h. eine senkrecht zur Symmetrieachse 9 ausgerichtete Ebene.

Die Figur 4 zeigt einen Ausschnitt der im Einschubrahmen 38 des Lichtvisiers 14 eingesetzten Skala 37. Man erkennt hier den für die Erzeugung des Lichtfächers verantwortlichen Spalt 36, die die Skalenstriche erzeugenden Querschlitze 37, die den Skalenstrichen entsprechen, sowie die den Skalenstrichen zugeordneten Zahlenangaben. Alle Zahlen und auch die Skala selbst sind zur Kompensation des unterschiedlichen, aus Figur 3 erkennbaren Objekt-Bildabstandes in Skalenlängsrichtung verzerrt, d. h. jene Teile der Skala und der Beschriftung, die näher zur Bildebene 24 angeordnet sind, sind größer und jene, die weiter von der Bildebene entfernt sind, kleiner dargestellt, damit sie sich in der Bildebene gleichmäßig abbilden (Die Konizität ist in der Figur 4 übertrieben dargestellt). Die einzelnen Skalenstriche haben untereinander nicht den gleichen, sondern einen mit steigendem Zahlenwert schrumpfenden Abstand.

Die Lichtquelle 32 des Lichtvisiers 14 beleuchtet über die Kondensorlinse 33, wie anhand der Figur 3 gezeigt werden kann, die Rückseite der Schablone 35. Die Durchbrüche der Schablone werden von der Objektlinse 34 auf den jeweiligen Bildebenen 24, 25, 26 abgebildet. Anhand der Punkte P1 und P2 wird der Strahlengang gezeigt, durch den diese Punkte auf die mittlere Bildebene 24 und den benachbarten Bildebenen abgebildet werden. Es ist zu beachten, daß die Punkte auf der darunterliegenden Ebene nach links und auf der darüberliegenden Ebene nach rechts verschoben sind. Die Zahlenwerte auf der Skala sind so gewählt, daß diejenigen Punkte der Skala, die sich in den einzelnen Bildebenen der Symmetrieachse der Strahlenblende schneiden, einen dem Abstand zum Fokus entsprechenden Zahlenwert tragen.

Da, wie anhand der Figur 2 deutlich wird, die einzelnen Skalenstriche die Symmetrieachse 9 der Strahlenblende 8 unter einem jeweils anderen Winkel schneiden, darf die Skala 37 nicht linear, sondern muß in einem entsprechenden Maße in der einen Richtung gedehnt sein. Wird nunmehr in den Strahlengang, d. h. zwischen dem Strahlerkopf 5 und dem Strahlenfänger 7 des Strahlentherapiegerätes 3, ein Objekt, beispielsweise ein Patient 10, auf einer Lagerungsplatte 11 liegend eingeführt, so wird beim Einschalten der beiden, der Patientenoberseite zugewandten Lichtvisiere 14 die diesen zugewandte Oberseite des Patienten angestrahlt. Wie die Darstellungen der Figuren 1 und 2 deutlich machen, schneiden sich die Lichtfächer 21, 22 dieser beiden Lichtvisiere abhängig vom Fokusabstand stets an jener Stelle der Hautoberfläche des Patienten 10, die auf der Symmetrieachse 9 der Strahlenblende 8 liegt. Das ist jene Stelle der Hautoberfläche, die bei maximaler Aufblendung der Strahlenblende vom Zentralstrahl getroffen würde.

Die von dem einen Lichtvisier 14 mitprojizierte Skala 37 ist in ihrer ganzen Länge nach auf der Hautoberfläche des Patienten sichtbar. Wie die Figur 3 aber deutlich macht, verschiebt sie sich wegen der schrägen Projektion zur Symmetrieachse 9 der Strahlenblende 8 in Längsrichtung, wenn sich die senkrecht zur Symmetrieachse ausgerichtete Oberfläche, auf der sie projiziert wird, in Richtung zur Strahlenquelle hin oder von ihr weg verschiebt. Durch entsprechende Dimensionierung der Skala oder/und durch Änderung des Winkels zwischen der Symmetrieachse 9 der Strahlenblende 8 und dem Lichtfächer 21, 22 des Lichtvisiers 14 kann erreicht werden, daß der im Schnittpunkt der beiden Lichtfächer ablesbare Skalenwert dem jeweiligen Fokusabstand entspricht.

Der untersuchende Arzt erkennt nun beim Einstellen des Strahlentherapiegerätes 3 mit Hilfe der Lichtvisiere 12, 13, 14 auf der Hautoberfläche des Patienten 10 den Auftreffpunkt des Zentralstrahls. Zugleich kann er im Koordinatenmittelpunkt, d. h. dem Schnittpunkt der beiden Lichtfächer 21, 22, den Skalenwert, d. h. den Fokusabstand der Hautoberfläche, ablesen. Er kann nun den Patienten 10 durch Drehen des Röntgenuntersuchungsgerätes 3 oder/und durch Verschieben der Patientenlagerungsplatte 11 zum Zentralstrahl zentrieren und den jeweils gewünschten Fokus-Haut-Abstand einstellen. Da der Abstand des Fokus von der Rotationsachse 2 stets bekannt ist, läßt sich durch Subtraktion der beiden Zahlen auch sofort ermitteln, wieviel Zentimeter unter der Hautoberfläche sich das Isozentrum 20 befindet. Dabei ist es der große Vorteil dieser Lichtvisieranordnung, daß kein materieller Körper, also auch kein Spiegel, in dem Strahlengang zwischen Strahlenquelle und Patient 10 erforderlich ist, so daß diese Lichtvisieranordnung von der gewählten Strahlenart des Strahlentherapiegerätes 3 unabhängig ist.

**Ansprüche**

1. Strahlentherapiegerät zur Bestrahlung von Patienten, mit einer Strahlenblende und mit Lichtvisieren für die Projektion von die Geräteeinstellung markierenden Koordinaten auf die Oberfläche des zu bestrahlenden Objektes und mit zwei je einen zweidimensionalen Lichtfächer projizierenden Lichtvisieren, die in der Weise am Strahlentherapiegerät befestigt sind, daß sich die Ebenen der beiden Lichtfächer längs der Symmetrieachse der Strahlenblende schneiden und die Projektionsrichtungen der Lichtfächer über den gesamten Öffnungswinkel eines jeden der beiden Lichtfächer hinweg einen Winkel sowohl mit der Symmetrieachse als auch mit senkrecht zur Symmetrieachse ausgerichteten Ebenen bilden, dadurch gekennzeichnet, daß eine Skala (37) mit dem einen der beiden Lichtfächer (21) mitprojiziert wird, die im jeweiligen Schnittpunkt der beiden Lichtfächer (21, 22) mit einer senkrecht zur Symmetrieachse ausgerichteten Ebene die Entfernung zum Fokus der Strahlenquelle des Strahlentherapiegerätes (3) bezeichnet.

2. Strahlentherapiegerät nach Anspruch 1, dadurch gekennzeichnet, daß die Ebenen der beiden Lichtfächer (21, 22) in einer senkrecht zur Symmetrieachse (9) der Strahlenblende (8) des Strahlentherapiegerätes (3) ausgerichteten Ebene (24, 25, 26) miteinander einen Winkel von mindestens 10°, höchstens jedoch 170° bilden.

3. Strahlentherapiegerät nach Anspruch 1, dadurch gekennzeichnet, daß die Projektionsrichtung der Lichtvisiere (12, 13, 14) über den gesamten Öffnungswinkel des jeweiligen Lichtfächers (21, 22) hinweg mit einer senkrecht zur Symmetrieachse (9) der Strahlenblende (8) ausgerichteten Ebene (24, 25, 26) einen Winkel von mindestens 20° bis höchstens 80° bilden.

4. Strahlentherapiegerät nach Anspruch 1, dadurch gekennzeichnet, daß die Ziffern der Skala (37) im Lichtvisier (12, 14) in der Weise verzerrt sind, daß sie erst durch die Projektion auf eine senkrecht zur Symmetrieachse (9) ausgerichtete Ebene (24, 25, 26) entzerrt werden.

5. Strahlentherapiegerät nach Anspruch 1, dadurch gekennzeichnet, daß die zu projizierenden Ziffern in der Objektebene des Lichtvisiers (12, 14) in der Weise konisch verzerrt sind, daß die der Bildebene (24) näher gelegene Seite der Ziffern in der Objektebene vergrößert, die der Bildebene ferner liegende Seite der Ziffern in der Objektebene verkleinert ist.

6. Strahlentherapiegerät nach Anspruch 1, dadurch gekennzeichnet, daß die Symmetrieachse (9) der maximal aufgeblendeten Strahlenblende (8) die Rotationsachse (2) des Strahlentherapiegerätes (3) schneidet.

7. Strahlentherapiegerät nach Anspruch 1, dadurch gekennzeichnet, daß die Symmetrieachse (9) die Rotationsachse (2) des Strahlentherapiegerätes (3) in einem rechten Winkel schneidet.

8. Strahlentherapiegerät nach Anspruch 1, dadurch gekennzeichnet, daß die Bestrahlung mit beschleunigten Elektronen erfolgt.

## Claims

1. Radiation therapy apparatus for the irradiation of patients, comprising a beam diaphragm and light visors for the projection onto the surface of the object to be irradiated of coordinates marking the device setting, and two light visors each of which projects a two-dimensional fan-shaped beam of light and which are attached to the radiation therapy apparatus in such manner that the planes of the two fan-shaped beams of light intersect along the axis of symmetry of the beam diaphragm and in such a manner that over the entire opening angle of each of the two fan-shaped beams of light the projection directions of the fan-shaped beams of light form an angle both with the axis of symmetry and with planes aligned at right angles to the axis of symmetry, characterised in that together with one of the two fan-shaped beams of light (21) a scale (37) is additionally projected which, at the relevant intersection point of the two fan-shaped beams of light (21, 22) with a plane aligned at right angles to the axis of symmetry, designates the distance to the focus of the radiation source of the radiation therapy apparatus (3).

2. Radiation therapy apparatus as claimed in Claim 1, characterised in that the planes of the two fan-shaped beams of light (21, 22) form a mutual angle of at least 10° but at the maximum 170° in a plane (24, 25, 26) aligned at right angles to the axis of symmetry (9) of the beam diaphragm (8) of the radiation therapy apparatus (3).

3. Radiation therapy apparatus as claimed in Claim 1, characterised in that over the entire opening angle of a given fan-shaped beam of light (21, 22), the projection direction of the light visors (12, 13, 14) forms an angle of at least 20° up to a maximum of 80° with a plane (24, 25, 26) aligned at right angles to the axis of symmetry (9) of the beam diaphragm (8).

4. Radiation therapy apparatus as claimed in Claim 1, characterised in that the digits of the scale (37) in the light visor (12, 14) are distorted in such manner that they are corrected only by projection onto a plane (24, 25, 26) aligned at right angles to the axis of symmetry (9).

5. Radiation therapy apparatus as claimed in Claim 1, characterised in that the digits to be projected are conically distorted in the object plane of the light visor (12, 14) in such manner that the side of the digits which is closer to the image plane (24) are enlarged in the object plane, whereas that side of the digits which is further removed from the image plane is reduced in size in the object plane.

6. Radiation therapy apparatus as claimed in Claim 1, characterised in that the axis of symmetry (9) of the maximum opening of the beam diaphragm (8) intersects the axis of rotation (2) of the radiation therapy apparatus (3).

7. Radiation therapy apparatus as claimed in Claim 1, characterised in that the axis of symmetry (9) intersects the axis of rotation (2) of the radiation therapy apparatus (3) at a right angle.

8. Radiation therapy apparatus as claimed in Claim 1, characterised in that the irradiation is carried out using accelerated electrons.

## Revendications

1. Appareil de thérapie par irradiation pour l'irradiation de patients, avec diaphragme et viseurs lumineux pour la projection des coordonnées marquant le réglage de l'appareil sur la surface de l'objet à irradier et avec deux viseurs lumineux projetant chacun une nappe lumineuse bidimensionnelle en éventail, lesquels viseurs sont fixés à l'appareil de thérapie par irradiation de manière que les plans des deux nappes lumineuses se coupent le long de l'axe de symétrie du diaphragme et que les directions de projection des nappes lumineuses forment, sur tout l'angle d'ouverture de chacune des deux nappes lumineuses, un angle aussi bien avec l'axe de symétrie qu'avec également le plan perpendiculaire à l'axe de symétrie, caractérisé en ce qu'on projette simultanément avec l'un des deux éventails lumineux (21), une échelle graduée (37) qui indique, au point d'intersection des deux éventails lumineux (21, 22) avec un plan dirigé perpendiculairement à l'axe de symétrie, la distance au foyer du générateur de l'appareil de thérapie par irradiation (3).

2. Appareil de thérapie par voie d'irradiation selon la revendication 1, caractérisé en ce que les plans des deux éventails lumineux (21 et 22) forment entre eux, dans un plan (24, 25 et 26) disposé perpendiculairement à l'axe de symétrie (9) du diaphragme (8) de l'appareil de thérapie par voie d'irradiation (3), un angle d'au moins 10°, mais au plus égal de 170°.

3. Appareil de thérapie par voie d'irradiation selon la revendication 1, caractérisé en ce que la direction de projection des viseurs lumineux (12, 13 et 14) forme, sur la totalité de l'angle d'ouverture de l'éventail lumineux concerné (21 et 22), avec un plan (24, 25 et 26) disposé perpendiculairement à l'axe de symétrie (9) du diaphragme (8), un angle d'au moins 20° à au plus 80°.

4. Appareil de thérapie par voie d'irradiation selon la revendication 1, caractérisé en ce que les chiffres de l'échelle (37) sont déformés dans le viseur lumineux (12, 14) de telle sorte qu'ils sont rétablis seulement par projection sur un plan (24, 25 et 26) disposé perpendiculairement, à l'axe de symétrie (9).

5. Appareil de thérapie par voie d'irradiation selon la revendication 1, caractérisé en ce que les chiffres à projeter dans le plan objet du viseur lumineux (12, 14) sont déformés coniquement de telle sorte que le côté des chiffres situé le plus près du plan image (24), est grossi dans le plan objet, alors que le côté des chiffres qui est éloigné plus du plan image est réduit dans le plan objet.

6. Appareil de thérapie par voie d'irradiation selon la revendication 1, caractérisé en ce que l'axe de symétrie (9) du diaphragme (8) ouvert au maximum coupe l'axe de rotation (2) de l'appareil de thérapie par voie d'irradiation (3).

7. Appareil de thérapie par voie d'irradiation selon la revendication 1, caractérisé en ce que l'axe de symétrie (9) coupe l'axe de rotation (2) de l'appareil de thérapie par voie d'irradiation (3) à angle droit.

8. Appareil de thérapie par voie d'irradiation selon la revendication 1, caractérisé en ce que l'irradiation est effectuée avec des électrons accélérés.

0 024 504

FIG 1

FIG 4

1

FIG 2

FIG 3